# EUROPEAN PATENT APPLICATION

(11) **EP 3 723 069 A1**
(43) Date of publication of application: **14.10.2020**
(21) Application number: 20168236.6
(22) Date of filing: 06.04.2020
(51) Int. Cl.: G09B 23/28, G09B 23/30

(54) **SYSTEMS AND METHODS FOR SIMULATING SURGICAL PROCEDURES**

(30) Priority: 08.04.2019 US 201962830605 P; 10.03.2020 US 202016813866
(71) Applicant: Covidien LP, Mansfield, MA 02048 (US)
(72) Inventor: ROSSETTO, Francesca, Longmont, CO 80504 (US); SARTOR, Joe D., Longmont, CO 80504 (US)
(74) Representative: Maschio & Soames IP Ltd

(57) **Abstract**

A system for simulating thoracoscopic lung surgery includes a surgical device and a display for displaying the simulated thoracoscopic lung surgery. The system predicts the effect the surgical device would have on actual tissue and displays a visual representation of the tissue and the effect a manipulation of the surgical device would have on the tissue.

## Description

### CROSS-REFERENCE TO RELATED APPLICATION

This application claims the benefit of and priority to U.S. Provisional Patent Application Serial No. 62/830,605, filed April 8, 2019, the entire disclosure of which is incorporated by reference herein.

### SUMMARY

To treat certain diseases of the lung, the diseased or malfunctioning lung tissue may be removed or resected. After resecting the subject lung tissue, a surgical instrument, such as a surgical stapler, an electrosurgical forceps, or the like, may be utilized to ligate the lung tissue and effectuate a seal. Sometimes, a physician may undergo training on these procedures by performing a simulated laparoscopic surgical procedure on either a live animal or ex-vivo tissue.

According to an aspect of the disclosure, a system for simulating thoracoscopic lung surgery is provided and includes a simulator and a workstation in electrical communication with the simulator. The workstation includes a display, a processor coupled to the display, and a memory coupled to the processor. The memory has instructions stored thereon which, when executed by the processor, cause the workstation to receive position information of a surgical device from the simulator, generate on the display a visual representation of the surgical device relative to a visual representation of an anatomical feature, and simulate, on the display, an effect a manipulation of the surgical device has on the visual representation of the anatomical feature.

In aspects, the system may further include an EM sensor associated with the surgical device. Receiving position information of the surgical device may include receiving position information from the EM sensor, and the position information may indicate a position of the surgical device in space.

In some aspects, the surgical device may be a working surgical device, a control representative of a working surgical device, or a virtual surgical device.

In further aspects, the workstation may predict the effect on the visual representation of the anatomical feature based on an analysis of the position information of the surgical device.

In other aspects, the workstation may predict the effect on the visual representation of the anatomical feature based on a type of actuation of the surgical device.

In aspects, the type of actuation of the surgical device may include clamping, stapling, and/or cutting.

In some aspects, simulating, on the display, the effect the manipulation of the surgical device has on the visual representation of the anatomical feature may include generating on the display a change in state of the visual representation of the anatomical feature.

In further aspects, the change in state of the visual representation of the anatomical feature may be displayed as a movement of a piece of virtual tissue of the visual representation of the anatomical feature.

In other aspects, the instructions stored on the memory, when executed by the processor, may cause the workstation to generate on the display a type of actuation of the surgical device.

In aspects, the system may further include a housing defining an internal volume representative of a thoracic cavity. The surgical device may be movably coupled to the housing.

In some aspects, the visual representation of the anatomical feature may be a generated model based on medical imaging data of the anatomical feature of a patient.

In further aspects, the medical imaging data may be computerized tomography (CT) scan data of the patient's anatomical feature.

In aspects, the patient's anatomical features may be segmented to assign specific tissue properties (e.g., density, elastic modulus, Poisson's ratio) as needed to perform deflection calculations of the entire organ or anatomic region, including collapse based on applied pressure or regional tissue and organ deflections based on locally induced virtual deflections from a surgical device.

In other aspects, they system may further include an imaging device configured to image the surgical device to gather the position information of the surgical device.

In aspects, the visual representation of the anatomical feature may include virtual tissue. The position information of the surgical device may be used to apply local displacements to the virtual tissue.

In some aspects, a reaction of the virtual tissue to the applied local displacement may be calculated from mechanical properties assigned to structures in the virtual tissue.

In further aspects, the mechanical properties may be assigned by tissue type. The tissue type may include parenchyma, vasculature, bronchi, tumor, cartilage, and muscle.

In another aspect of the disclosure, a system for simulating thoracoscopic lung surgery is provided and includes a surgical device, an imaging device configured to capture images including a portion of the surgical device, and a workstation in electrical communication with the surgical device and/or the imaging device. The workstation includes a display, a processor coupled to the display, and a memory coupled to the processor. The memory has instructions stored thereon which, when executed by the processor, cause the workstation to receive image data from the imaging device, analyze the image data to determine position information of the surgical device, generate on the display a visual representation of the surgical device relative to a visual representation of an anatomical feature based on the determined position information of the surgical device, and simulate, on the display, an effect a manipulation of the surgical device has on the visual representation of the anatomical feature.

In aspects, the workstation may predict the effect on the visual representation of the anatomical feature based on an analysis of the position information of the surgical device.

In some aspects, the workstation may predict the effect on the visual representation of the anatomical feature based on a type of actuation of the surgical device.

In further aspects, simulating, on the display, the effect the manipulation of the surgical device has on the visual representation of the anatomical feature may include generating on the display a change in state of the visual representation of the anatomical feature based on displacement of the surgical device and tissue properties being acted on by the surgical device.

In aspects, the surgical device may be a virtual representation of a surgical device.

In yet another aspect of the disclosure, a method of simulating thoracoscopic lung surgery is provided and includes receiving position information of the surgical device, generating on the display a visual representation of the surgical device relative to a visual representation of an anatomical feature, predicting an effect a manipulation of the surgical device would have on the anatomical feature, and generating on the display a change in state of the visual representation of the anatomical feature. The change in state may correspond to the predicted effect on the anatomical feature.

In aspects, the method may further include displaying on the display a movement of a piece of virtual tissue of the visual representation of the anatomical feature.

In some aspects, the method may further include generating on the display a type of actuation of the surgical device.

### BRIEF DESCRIPTION OF THE DRAWINGS

The accompanying drawings, which are incorporated in and constitute a part of the specification, illustrate embodiments of the disclosure and, together with a general description of the disclosure given above as well as the detailed description of the embodiment or embodiments given below, serve to explain the principles of this disclosure.
FIG. 1 is a schematic diagram of a laparoscopic training system in accordance with aspects of the disclosure;
FIG. 2 is a schematic block diagram of an illustrative embodiment of a computing device that may be employed in various aspects of the system or components of FIG. 1;
FIG. 3 is a flowchart showing a first illustrative method for training a clinician to perform a surgical procedure with the laparoscopic training system shown in FIG. 1;
FIG. 4 depicts an exemplary user interface that may be presented on the display of the training system of FIG. 1, including simulated images of a lung;
FIG. 5A is a front perspective view of a computer generated model of components of a respiratory system including a trachea and left and right lungs; and
FIG. 5B is a front perspective view of the computer generated model shown in FIG. 5A with the blood vessels removed from the left lung to better illustrate a lesion in the left lung.

### DETAILED DESCRIPTION

Simulated surgical procedures for training purposes are traditionally performed on either a live animal or ex-vivo tissue (e.g., harvested organs such as a bovine or pig lung, liver, etc.). Prior to training, the tools are set-up in a training surgical suite or an operational surgical suite, sometimes a working suite taken out of service. The use of industry training facilities adds additional costs such as maintenance of the facility and transportation of personnel and/or equipment to and from the facility. Once training has finished, placing the operational surgical suite back in service requires sterilization and replacement of suite equipment. Known systems and methods of training which include the use of live animals or ex-vivo tissue additionally require disposal of biological waste.

Accordingly, there is a continuing need for improved simulation visualization techniques used in laparoscopic surgical procedure training. In particular, while new commercial systems generally make simulating the treatment of tissue easier (particularly laparoscopic procedures), these systems generally rely on simplified artistic images or video imaging of a surgical site which may or may not represent a particular organ or anatomical feature with the desired level of detail.

As such, the disclosure presents clinicians with training systems capable of more realistically simulating laparoscopic surgeries without having to use ex-vivo tissue or live animals. The training systems include a workstation (e.g., a computer and a display) and a simulator (e.g., one or more surgical devices operably coupled to a housing defining an internal space or a virtual representation of one or more surgical devices). The workstation receives signals from a laparoscopic surgical device (inoperable or fully operable) or a control that simulates a working surgical device, and a position tracker associated with the surgical device for tracking a position of the surgical device during its use. The surgical device or a virtual representation of a surgical device is mapped on the display of the workstation over an actual patient anatomy reconstructed from CT, PET, or MRI data, whereby the simulated surgical procedure being performed is displayed as if the patient from which the imaging data is taken was being operated on rather than the internal space of the housing. In other aspects, instead of displaying a patient's anatomy taken from actual imaging data, a pre-set anatomy (e.g., a simulation of a collapsed lung within a thoracic cavity) may be displayed on the display.

In embodiments, signals may be received by the workstation from known imaging devices, such as computed tomography (CT) imaging devices, cone-beam CT imaging devices, magnetic resonance imaging (MRI) devices, and fluoroscopy imaging devices, which indicate the position of the respective surgical device and/or imaging device in three-dimensional space. For purposes of clarity, reference will be made to systems incorporating visual imaging devices, though it is contemplated that any of the above-mentioned imaging systems may be simulated during simulated procedures.

Signals may be received by the workstation from an imaging device. Based on the signals received by the workstation from the imaging device, visual and/or audio feedback may be generated by the workstation (e.g., two-dimensional (2D) or three-dimensional (3D) images, a 2D or 3D video stream, and/or audible tones). In some aspects, the housing may be a phantom including synthetic tissue mass (e.g., a synthetic liver, synthetic torso, and the like). The phantom may simulate the function of a chest cavity by being transitionable between contracted and expanded states, and may be equipped with rib-like structures (not shown) to enhance the lifelike appearance of the phantom.

During simulated surgeries, a clinician may manipulate a working surgical device, a replica of a surgical device, or a hand-control that simulates a working surgical device. The workstation and simulator, as well as the associated components thereof, may be directly or indirectly in electrical communication (either via wired or wireless connection) with the workstation, or to one another.

During a simulated surgical procedure, the clinician causes the surgical device and the imaging device to be passed through ports along the exterior surface of a housing. The simulator may include an electromagnetic (EM) field generator forming part of an EM tracking system which tracks the position and orientation (also commonly referred to as the "pose") of EM sensors disposed on the surgical device and the imaging device. Additionally, or alternatively, the simulator may include an imaging device located away from the simulator, the imaging device configured to capture images of the simulator when acted upon by a clinician with the surgical device and the imaging device for the purpose of tracking the devices in space. The simulator then transmits the information received by the EM tracking system and/or the imaging device to the workstation, which, in turn, determines the pose of the instruments in three-dimensional space. In embodiments, inertial measurement units (IMUs) including accelerometers and/or gyroscopes, acoustic tracking, as well as other known tracking systems and sensors may be used for detecting and determining the pose of the surgical imaging instruments and/or the surgical devices.

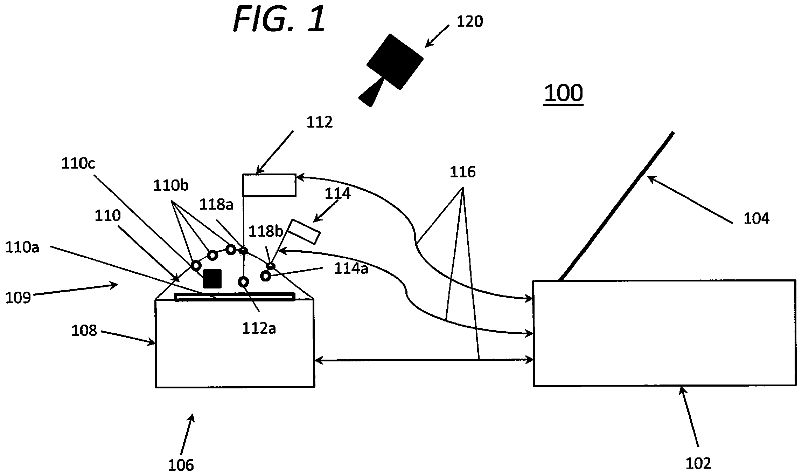

FIG. 1 illustrates a schematic diagram of a laparoscopic surgical training system 100 configured to be used by one or more clinicians during a simulated surgical procedure to enable performance of a video assisted surgical procedure ("VATS"). The training system 100 includes an operation simulator 106 in communication with a workstation 102 (via either wired or wireless communication). The operation simulator 106 includes a laparoscopic surgical device 112, a laparoscopic imaging device 114 coupled to the surgical device 112, and a housing 110. The surgical device 112 and the imaging device 114 may be coupled to the workstation 102 via one or more wired or wireless connections 116.

The simulator 106 includes a base 108 having the housing 110 disposed thereon. The base 108 may include connectivity ports (not explicitly shown) which couple to the connections 116 associated with the surgical device 112, the imaging device 114, and/or the workstation 102. The housing 110 supports the surgical device 112 (e.g., an actual surgical device or a control knob, glove, mouse, or the like manipulatable in a similar manner as an actual surgical device) and an imaging device (e.g., a video imaging device configured to image an interior portion of the body of a patient) thereon. In aspects, the surgical device may be a virtual surgical device displayed and manipulatable on a display 104. The housing 110 may be formed in the shape of a lung (in a normal or collapsed configuration) to approximate corresponding visual representations of the internal structure of the anatomic feature displayed by the workstation 102, which anatomically approximate living organs. The housing 110 may further include a bellow 110c (FIG. 1) or other such suitable components capable of manipulating the housing 110 (e.g., expanding and contracting the exterior surface of the housing 110) which, during operation, cause the housing 110 to move during simulated surgeries. The housing 110 may be equipped with force sensors positioned at selected locations within the housing 110 and in communication with the workstation 110. The force sensors transmit data (e.g., forces exerted on the housing 110 by the surgical devices 112). The housing 110 further includes ports 118a, 118b, configured to receive the surgical device 112 and the imaging device 114, respectively. The ports 118a, 118b enable distal portions of the surgical device 112 and the imaging device 114 to pass through a surface of the housing 110 as would traditionally occur during a typical laparoscopic surgical procedure. The ports 118a, 118b may be rigid or semi-rigid, to represent the elasticity of the tissue of a patient.

An EM field generator 110a may be disposed either in or on the base 108 or beneath the housing 110 so as to generate an EM field for capturing the position of one or more EM sensors in proximity to, or disposed on, the simulator 106. The housing 110 may also have one or more EM reference sensors 110b disposed either internal or external to the housing 110 which capture the pose of the housing 110 intermittently or continuously during the simulated surgical procedure. In response to the generation of the EM field, a tracking module (not explicitly shown) may receive signals from each of the EM reference sensors 110b, 112a, 114a and, based on the signals, derive the location of each EM reference sensor 110b, 112a, 114a, as well as their position along the device to which they are coupled in six degrees of freedom. In addition, one or more reference sensors may be disposed in fixed relation to the housing 110. Signals transmitted by the reference sensors to the tracking module may subsequently be used to calculate a patient coordinate frame of reference. Registration is generally performed by identifying select locations in both the stored representation of the anatomical feature associated with the housing 110 and the reference sensors disposed along the housing 110.

A surgical device EM sensor 112a and an imaging device EM sensor 114a are disposed on the surgical device 112 and the imaging device 114, respectively. Additionally, the surgical device EM sensor 112a and the imaging device EM sensor 114a may include an array of EM sensors (not explicitly shown) disposed along the respective device in a predetermined pattern, so as to provide a more accurate positional measurement of the device. Collectively, the EM components disclosed herein will be referred to as the EM tracking system 109.

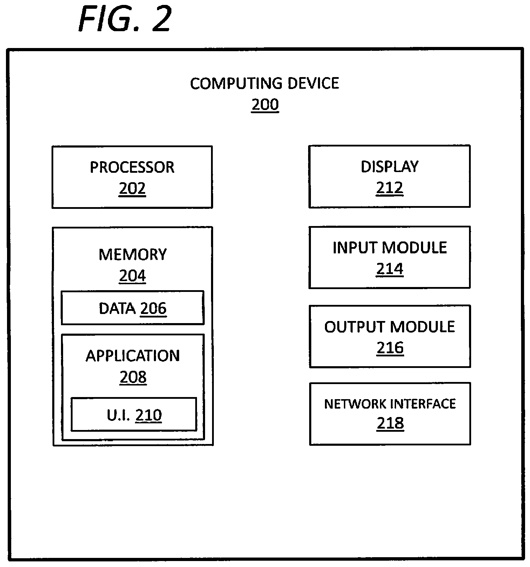

With reference to FIGS. 1 and 2, the workstation 102 of the training system 100 may have training software stored as an application 208 in a memory 204 of a computing device 200. The workstation 102 may have additional software or instructions stored therein which may be executed while the workstation 102 is in use. When the application 208 is executed by the processor 202 of the workstation 102, the application may control a display 104 of the workstation 102 to cause the display 104 to output one or more visual and/or audible outputs (e.g., a series of images, a 2D or 3D video stream, or sound to speakers integrated into the display 104 (not explicitly shown)). The images to be displayed may include, without limitation, ultrasound images, simulated ultrasound images, CT images, simulated CT images, 3D models, and other predetermined user-interfaces for simulating video assisted thoracic surgeries. The visual and/or audible output may be transmitted by the workstation 102 for display on the display 104 in response to input, such as positional data and/or a device state or configuration of either the surgical device 112 and/or the imaging device 114.

The computing device 200, or one or more components thereof, may represent one or more components (e.g., workstation 102, simulator 106, surgical device 112, simulated imaging device 114, etc.) of the training system 100. The computing device 200 may include one or more processors 202, memories 204, display devices or displays 212, input modules, 214, output modules 216, and/or network interfaces 218, or any suitable subset of components thereof. The memory 204 includes non-transitory computer readable storage media for storing data and/or software having instructions that may be executed by the one or more processors 202 and which, when executed, control operation of the computing device 200, as well as various other devices in communication with the computing device 200. The memory 204 stores data 206 and/or one or more applications 208. Such applications 208 may include instructions which are executed on the one or more processors 202 of the computing device 200. In aspects, the application 208 may include instructions which cause a user interface component 210 to control the display 212 such that a user interface 210 is displayed (e.g., a graphical user interface (GUI)).

The workstation 102 may display multiple views such as, for example, a pre-scanned CT image and a simulated CT image on the display 104 of the workstation 102 to assist the clinician during the performance of a simulated surgical procedure. In addition to image data generated based on CT image data as well as simulated imaging device data, the workstation 102 may display navigational aids or visual cues, surgery specific data, information input during pre-operative planning (e.g., directions to a target area of tissue where a growth targeted for treatment is located), and the like.

The workstation 102 may, similar to the simulated surgical device 112 and the simulated imaging device 114, be in either wired or wireless electrical communication via a connection 116 with the simulator 106. While the surgical device 112 and the imaging device 114 are shown as connected to the workstation 102 via connections 116, the surgical device 112 and the imaging device 114 may be operably coupled to the workstation 102 via connection to the simulator 106. The simulator 106 may include one or more applications 208 stored in the memory 204 of the simulator 106 which, when executed on the processor 202 of the simulator 106, control the transmission of data to or from the simulator 106 to the workstation 102. Likewise, the workstation 102 may be integrated, either in whole or in part, into the simulator 106 such that the simulator 106 displays outputs similar to those described above during the simulated surgical procedures.

During operation, the EM tracking system 109 transmits signals to the workstation 102 to indicate the pose of any one of the EM reference sensors 110b, the surgical device EM sensor 112a, and the imaging device EM sensor 114a. The workstation 102, in response to receiving signals from the EM tracking system 109, determines a pose for each of the instruments associated with particular EM sensors. The EM tracking system 109 may measure or determine the position of any of the included instruments within three-dimensional space and further within proximity of the EM field generator 110a, thereby enabling the EM tracking system 109 to determine the position and orientation of the relevant components to the internal space within the housing 110 during the simulated surgical procedure.

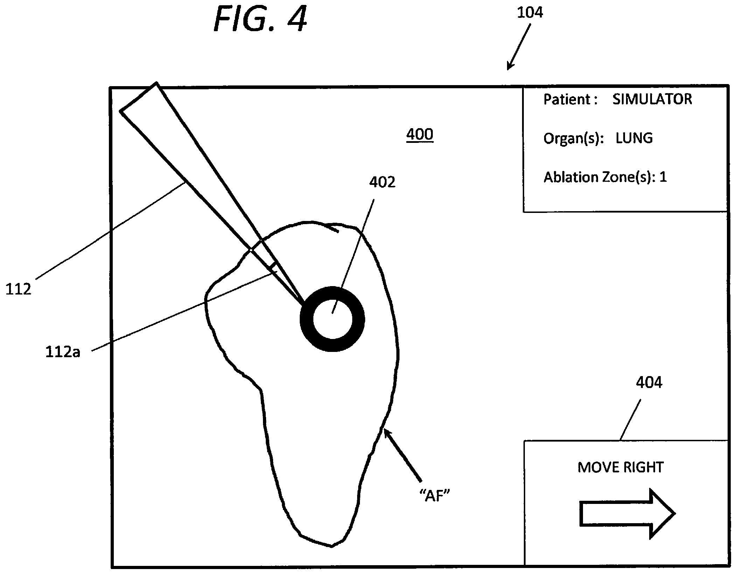

During simulated surgical procedures, the workstation 102 displays a series of images or video stream of the surgical site and/or CT images on the display 104, similar to those expected during a typical surgical procedure. For example, based on the determined pose of the housing 110, the surgical device 112, and the imaging device 114 relative to one another, a simulated surgical application 208 may display the position of the distal portion of the surgical device 112 relative to a visual representation of an anatomic feature "AF" (FIG. 4). The application 208 may simulate the various phases of a surgical procedure, including the generation of one or more 3D models during a planning phase or during the simulated surgical procedure, (e.g., identifying target locations and planning a pathway to the target locations as well as surgical interventions such as tissue resection to be performed), registering either stored or generated 3D models (e.g., calibrating the simulator for use with a phantom lung), navigation during a simulated surgical procedure to the target location or locations, performance of the planned surgical intervention at the target location, and the like. Models of anatomical features may be generated and stored either in a library of standard models (which include an average representation of an anatomical feature). Alternatively, if pre-operative scan data is available such as CT, magnetic resonance imaging (MRI), X-ray, cone beam computed tomography (CBCT), and/or positron emission tomography (PET) scan data, 3D models may be generated by the workstation 102 prior to or during a simulated surgical procedure so as to simulate a surgical procedure on the scanned anatomical feature.

During simulated surgical procedures, the application 208 may cause the display 104 to illustrate the position of the distal portion or distal tip of the surgical device 112 (e.g., a surgical stapler) relative to the target location 402 (FIG. 4) of an anatomical feature "AF" as would be illustrated during typical percutaneous and/or subcutaneous procedures. For example, to avoid providing clinicians with latent or otherwise undesired indication of the position of the surgical device 112 or other surgical instruments relative to the target location, the workstation 102 may continuously superimpose the position of the surgical device 112 onto a 3D model of the visual representation of the anatomical feature. By superimposing the position of the surgical device 112 onto the 3D model, the visual representation of the anatomical feature as well as the position of the surgical device 112 relative to the visual representation of the anatomical feature may be updated in the memory 204 and on the display 104 of the workstation 102 without reflecting any gaps, or other imperfections in the sensor data associated with the visual representation anatomical feature and/or the simulated surgical device 112. Where gaps become too great (e.g., a positional signal is not received for a predetermined period), the application 208 may cause the display 104 of the workstation 102 to display an alert (e.g., "SIGNAL ERROR") to warn the clinician that such a condition exists. Similarly, during a simulated surgical procedure, the application 208 may simulate such conditions (e.g., signal loss, signal errors, etc.) and cause the display to output information indicating such conditions.

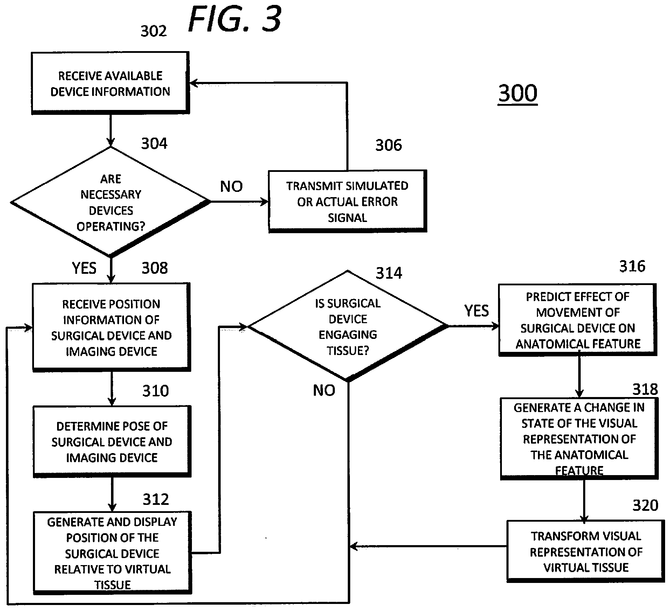

FIG. 3 illustrates a flowchart depicting an illustrative method 300 for simulating surgical procedures with the training system 100 (FIG. 1). The method 300 and associated techniques described herein enable visual simulation of a simulated surgical procedure via the display 104 of the training system 100 (FIG. 1). While the method 300 is described with reference to a particular sequence of steps, it will be apparent to one skilled in the art that certain steps described may be concurrently executed, or executed out of the sequence explicitly disclosed herein, without departing from the scope of the disclosure. The simulated procedure may begin with the workstation 102 receiving information from devices (e.g., the simulator 106, the surgical device 112, and the imaging device 114) such as a device ID or other information to identify the devices, as well as the operational state of the devices (e.g., operational, low battery, non-functional, etc.) (block 302). Once the workstation 102 receives the device information from the connected devices, the workstation 102 determines whether the necessary devices for performing the simulated procedure are connected and operational (block 304). If any of the devices in communication with the workstation 102 indicate that either they are non-functional or not ready to be used to perform the simulated surgical procedure, the workstation 102 causes the display 104 to output a relevant error message, including a message that certain devices are not connected, or are not operating properly (block 306). The method 300 may reiterate this process until it is determined that the training system 100 is ready for use.

When the workstation 102 determines that the appropriate devices are present, the method 300 continues and the workstation 102 receives information on the position of the surgical device 112 and the imaging device 114 relative to one another (block 308). More particularly, as discussed above, the EM tracking system 109 may capture signals from the EM reference sensors 110b, the surgical device EM sensor 112a, and the imaging device EM sensor 114a based on operation of the EM tracking system 109, thereby indicating the position of the surgical device 112 and imaging device 114 relative to the EM field generator 110a. Based on the position information, the workstation 102 may determine the pose of the surgical device 112 and/or the imaging device 114 (block 310).

The workstation 102 may receive sensor information from any of the earlier instrument tracking systems mentioned or an imaging device 120. Images captured by the imaging device 120 may capture optical and/or depth image data which, when transmitted to the workstation 102, enable the workstation 102 to determine the position of the surgical device 112 and/or the imaging device 114 relative to one another (see block 308). For example, one or more optical imaging sensors and/or infrared (IR) or depth sensors may be positioned to image the simulator 106 as well as devices engaging the simulator 106 during simulated surgical procedures. The optical imaging sensors, IR sensors, or depth sensors, may identify the pose of the surgical device 112 and/or the imaging device 114 and, based on the identification, transmit sensor signals to the workstation 102 indicative of the pose of the surgical device 112 and/or the imaging device 114 in three-dimensional space.

Imaging devices (e.g., a portable CT imaging device) may capture position-identifying information such as, without limitation, markers disposed about the housing 110, the surgical device 112, and/or the imaging device 114. The imaging devices may then transmit the captured image information to the workstation 102 which registers the position of the markers, and their respective device, to determine the pose of each device in three-dimensional space.

The workstation 102 generates an image or images to be displayed on the display 104 indicative of the positions of the surgical device 112 and the imaging device 114 (block 312). The visual representation of the anatomical feature displayed on the display 104 is generated and displayed relative to a visual representation of a pre-selected starting position of the surgical device 112. In a simulated surgical procedure, when a clinician manipulates the surgical device 112 from the starting position to another position determined using the EM sensors 110b, 112a, 114a and/or the imaging device 114, the workstation 102 depicts on the display 104 a movement of the image of the surgical device 112 relative to the image of the anatomical feature based on the determined change in position and/or orientation of the surgical device 112 from the starting position. The visual representation of the surgical device 112 and the anatomical feature may be shown from the point of view of the imaging device 114.

If the workstation 102 determines that the surgical device 112 has been moved to a position in which the virtual surgical device 112 engages virtual tissue of the visual representation of the anatomical feature ("YES" at block 314), the workstation 102 predicts an effect the surgical device 112 would have on the anatomical feature (block 316). The workstation 102 predicts the effect based on known characteristics of the actual tissue being represented on the display 104 and based on the determined force with which the surgical device 112 is moved, the determined direction the surgical device 112 is moved in, the distance the surgical device 112 is moved. Other factors may be considered in predicting the effect, such as, for example, the speed of the surgical device 112, vibrations 112 of the surgical device 112, or the like. Based on the predicted effect, the workstation 102 generates on the display 104 a change in state of the visual representation of the anatomical feature (block 318). In particular, the virtual tissue is shown being moved in a manner as would have occurred if actual tissue were being engaged by the surgical device 112. Alternatively, if the workstation 102 determines that the anatomical representation was not engaged ("NO" at block 314), process 300 returns to block 308.

If the workstation 102 determines that the surgical device 112 is not positioned or has not, over the course of the simulated procedure, been advanced to a target site, the workstation 102 may overlay elements onto the generated display such as navigational aid 404 (FIG. 4) which assists the clinician in advancing the surgical device 112 to the target site. The navigational aid 404 may be displayed until the surgical device 112 is in position, e.g., is at a target region.

The clinician may input information which is received by the surgical device 112 that is subsequently transmitted to the workstation 102. The information received by the surgical device 112 may include selection of a power setting for electrical cutting of tissue during simulated surgeries, selection of a stapler configuration (e.g., switching between grabbing or cutting), or other known setting for a particular surgical device normally adjustable by the clinician during surgical procedures.

If the surgical device 112 is actuated by the clinician, the workstation 102 may generate images illustrating a transformation of the anatomical feature (block 320). For example, as the user actuates the surgical device 112 to effect treatment on the virtual tissue, the workstation 102 may generate images to visually represent the type of actuation of the surgical device 112 (e.g., cutting, ablating, stapling, etc.), and visually represent the effect the actuation has on the virtual tissue. In particular, the virtual tissue may be displayed as being cut, scored, ablated, or stapled depending on the type of actuation of the surgical device 112. In this way, any determined effect the actions of the surgical device 112 have on the virtual tissue will be illustrated on the display 104 as happening to the anatomy shown on the display 104 (e.g., an image of a lung taken from a CT scan). For example, if the system 100 determines that the surgical device 112 is pulling on a location of the virtual tissue, the image of the lung shown on the display 104 will be illustrated as being pulled at the corresponding location. Once the images are generated, process 300 may be repeated by returning to block 308 and advancing the surgical device 112 to a different target site.

FIG. 4 illustrates a user interface which may be displayed on the display 104 (FIG. 1) of the workstation 102 during simulated procedures. FIG. 4 shows an image generated by the laparoscopic surgical training system 100 and, more specifically, a visual representation of a distal portion of the surgical device 112 (e.g., a surgical stapler) imaged within the thoracic cavity of a patient with the distal portion of the surgical device 112 displayed as if imaged by an imaging device within the thoracic cavity.

In some embodiments, the system 100 may provide tactile feedback to the clinician as the clinician manipulates the surgical device in the simulated space. The tactile feedback simulates a predicted resistance to movement of the surgical device as if the surgical device were encountering actual tissue.

In aspects, processor 202 may manipulate the virtual tissue by applying a deflection to the virtual model using computational mechanics (finite element simulation) based on instrument tracking.

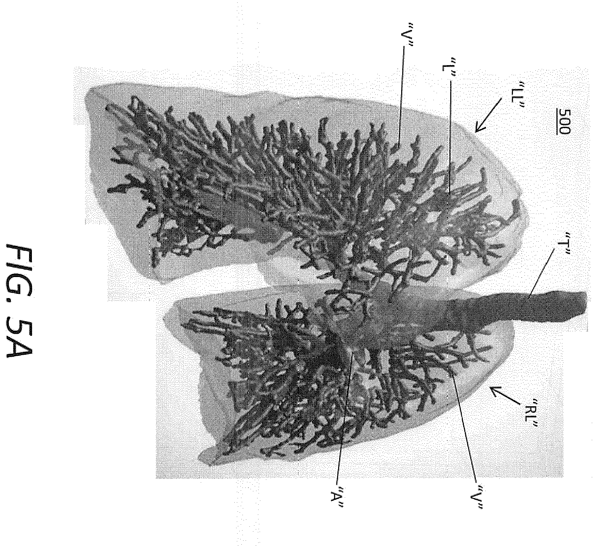

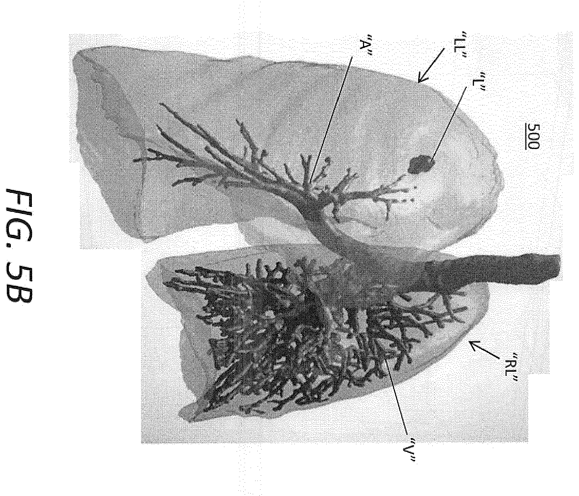

FIGS. 5A and 5B illustrate an exemplary computer generated model 500 of parts of a respiratory system for display by the workstation 102. The model 500 includes a trachea "T" and left and right lungs "LL," "RL." The model 500 also shows airways "A" (e.g., bronchi) and blood vessels "V" within the lungs "LL," "RL." Also provided in the model 500 is a lesion "L" shown in the upper left quadrant of the left lung "LL."

During a simulated surgical procedure, a user may actuate the surgical device 112 (FIG. 1) to effect treatment on virtual tissue, whereby the workstation 102 may visually represent the effect the actuation of the surgical device 112 has on the model 500. In this way, any determined effect the actions of the surgical device 112 would have on tissue may be shown on the display 104 by depicting the determined effect on the model 500 shown on the display 104. For example, if the system 100 determines that the surgical device 112 is pulling on virtual lung tissue, e.g., the right lung "RL," the appropriate portion of the right lung "RL" of the model 500 shown on the display 104 will be depicted as being pulled. Similarly, if the system 100 determines that the user intended to cut a portion of virtual lung tissue, e.g., the left lung "LL," the portion of the left lung "LL" of the model 500 shown on the display 104 will be depicted as being cut.

The term "clinician" refers to doctors, nurses, or other such support personnel that may participate in the use of the simulation systems disclosed herein; as is traditional, the term "proximal" refers to the portion of a device or component which is closer to the clinician whereas the term "distal" refers to the portion of the device or component which is further from the clinician. In addition, terms such as front, rear, upper, lower, top, bottom, and other such directional terms are used to aid in the description of the disclosed embodiments and are not intended to limit the disclosure. Well-known functions or constructions are not described in detail so as to avoid obscuring the disclosure unnecessarily.

While detailed embodiments of devices, systems incorporating such devices, and methods of using the same are described herein, these embodiments are merely examples of the subject-matter of the disclosure, which may be embodied in various forms. Therefore, specifically disclosed structural and functional details are not to be interpreted as limiting, but merely as providing a basis for the claims and as a representative basis for allowing one skilled in the art to variously employ the disclosure in appropriately detailed structure. Those skilled in the art will realize that the same or similar devices, systems, and methods as those disclosed may be used in other lumen networks, such as, for example, the vascular, lymphatic, and/or gastrointestinal networks as well. Additionally, the same or similar methods as those described herein may be applied to navigating in other parts of the body, such as the chest areas outside of the lungs, the abdomen, pelvis, joint space, brain, spine, etc.

The embodiments disclosed herein are examples of the disclosure and may be embodied in various forms. For instance, although certain embodiments are described as separate embodiments, each of the embodiments disclosed may be combined with one or more of the other disclosed embodiments. Similarly, references throughout the disclosure relating to differing or alternative embodiments may each refer to one or more of the same or different embodiments in accordance with the disclosure.

Any of the herein described methods, programs, algorithms or codes may be converted to, or expressed in, a programming language or computer program. The terms "programming language" and "computer program," as used herein, each include any language used to specify instructions to a computer, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

Any of the herein described methods, programs, algorithms or codes may be contained on one or more machine-readable media or memory. The term "memory" may include a mechanism that provides (e.g., stores and/or transmits) information in a form readable by a machine such a processor, computer, or a digital processing device. For example, a memory may include a read only memory (ROM), random access memory (RAM), magnetic disk storage media, optical storage media, flash memory devices, or any other volatile or non-volatile memory storage device. Code or instructions contained thereon can be represented by carrier wave signals, infrared signals, digital signals, and by other like signals. It should be understood that the foregoing description is only illustrative of the disclosure. Various alternatives and modifications can be devised by those skilled in the art without departing from the disclosure. Accordingly, the disclosure is intended to embrace all such alternatives, modifications and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure.

The invention may be described by reference to the following numbered paragraphs:-
1. A system for simulating thoracoscopic lung surgery, the system comprising:
   a simulator; and
   a workstation in electrical communication with the simulator and including:
      a display;
      a processor coupled to the display; and
      a memory coupled to the processor, the memory having instructions stored thereon which, when executed by the processor, cause the workstation to:
         receive position information of a surgical device from the simulator;
         generate on the display a visual representation of the surgical device relative to a visual representation of an anatomical feature; and
         simulate, on the display, an effect a manipulation of the surgical device has on the visual representation of the anatomical feature.
2. The system according to paragraph 1, further comprising an EM sensor associated with the surgical device, wherein receiving position information of the surgical device includes receiving position information from the EM sensor, the position information indicating a position of the surgical device in space.
3. The system according to paragraph 1, wherein the surgical device is a working surgical device, a control representative of a working surgical device, or a virtual surgical device.
4. The system according to paragraph 1, wherein the workstation predicts the effect on the visual representation of the anatomical feature based on an analysis of the position information of the surgical device.
5. The system according to paragraph 4, wherein the workstation predicts the effect on the visual representation of the anatomical feature based on a type of actuation of the surgical device.
6. The system according to paragraph 5, wherein the type of actuation of the surgical device includes at least one of clamping, stapling, or cutting.
7. The system according to paragraph 1, wherein simulating, on the display, the effect the manipulation of the surgical device has on the visual representation of the anatomical feature includes generating on the display a change in state of the visual representation of the anatomical feature.
8. The system according to paragraph 7, wherein the change in state of the visual representation of the anatomical feature is displayed as a movement of a piece of virtual tissue of the visual representation of the anatomical feature.
9. The system according to paragraph 1, wherein the instructions stored on the memory, when executed by the processor, cause the workstation to generate on the display a type of actuation of the surgical device.
10. The system according to paragraph 1, wherein the simulator includes a housing defining an internal volume representative of a thoracic cavity, the surgical device movably coupled to the housing.
11. The system according to paragraph 1, wherein the visual representation of the anatomical feature is a generated model based on medical imaging data of the anatomical feature of a patient.
12. The system according to paragraph 1, wherein the visual representation of the anatomical feature includes virtual tissue, and the position information of the surgical device is used to apply local displacements to the virtual tissue.
13. The system according to paragraph 12, wherein a reaction of the virtual tissue to the applied local displacement is calculated from mechanical properties assigned to structures in the virtual tissue.
14. The system according to paragraph 13, wherein the mechanical properties are assigned by tissue type, the tissue type including parenchyma, vasculature, bronchi, tumor, cartilage, and muscle.
15. A system for simulating thoracoscopic lung surgery, the system comprising:
   a surgical device;
   an imaging device configured to capture images including at least a portion of the surgical device; and
   a workstation in electrical communication with at least one of the surgical device or the imaging device, the workstation including:
      a display;
      a processor coupled to the display; and
      a memory coupled to the processor, the memory having instructions stored thereon which, when executed by the processor, cause the workstation to:
         receive image data from the imaging device;
         analyze the image data to determine position information of the surgical device;
         generate on the display a visual representation of the surgical device relative to a visual representation of an anatomical feature based on the determined position information of the surgical device; and
         simulate, on the display, an effect a manipulation of the surgical device has on the visual representation of the anatomical feature.
16. The system according to paragraph 15, wherein the workstation predicts the effect on the visual representation of the anatomical feature based on an analysis of the position information of the surgical device.
17. The system according to paragraph 15, wherein the workstation predicts the effect on the visual representation of the anatomical feature based on a type of actuation of the surgical device.
18. The system according to paragraph 15, wherein simulating, on the display, the effect the manipulation of the surgical device has on the visual representation of the anatomical feature includes generating on the display a change in state of the visual representation of the anatomical feature.
19. The system according to paragraph 15, wherein the surgical device is a virtual representation of a surgical device.
20. A method of simulating thoracoscopic lung surgery, the method comprising:
   receiving position information of a surgical device;
   generating on the display a visual representation of the surgical device relative to a visual representation of an anatomical feature;
   predicting an effect a manipulation of the surgical device would have on the anatomical feature; and
   generating on the display a change in state of the visual representation of the anatomical feature, the change in state corresponding to the predicted effect on the anatomical feature.

## Claims

1. A system for simulating thoracoscopic lung surgery, the system comprising:
a simulator; and
a workstation in electrical communication with the simulator and including:
a display;
a processor coupled to the display; and
a memory coupled to the processor, the memory having instructions stored thereon which, when executed by the processor, cause the workstation to:
receive position information of a surgical device from the simulator;
generate on the display a visual representation of the surgical device relative to a visual representation of an anatomical feature; and
simulate, on the display, an effect a manipulation of the surgical device has on the visual representation of the anatomical feature.

2. The system according to claim 1, further comprising an EM sensor associated with the surgical device, wherein receiving position information of the surgical device includes receiving position information from the EM sensor, the position information indicating a position of the surgical device in space.

3. The system according to claim 1 or claim 2, wherein the surgical device is a working surgical device, a control representative of a working surgical device, or a virtual surgical device.

4. The system according to any preceding claim, wherein the workstation predicts the effect on the visual representation of the anatomical feature based on an analysis of the position information of the surgical device; preferably wherein the workstation predicts the effect on the visual representation of the anatomical feature based on a type of actuation of the surgical device.

5. The system according to claim 4, wherein the type of actuation of the surgical device includes at least one of clamping, stapling, or cutting.

6. The system according to any preceding claim, wherein simulating, on the display, the effect the manipulation of the surgical device has on the visual representation of the anatomical feature includes generating on the display a change in state of the visual representation of the anatomical feature; preferably wherein the change in state of the visual representation of the anatomical feature is displayed as a movement of a piece of virtual tissue of the visual representation of the anatomical feature.

7. The system according to any preceding claim, wherein the instructions stored on the memory, when executed by the processor, cause the workstation to generate on the display a type of actuation of the surgical device; and/or wherein the simulator includes a housing defining an internal volume representative of a thoracic cavity, the surgical device movably coupled to the housing.

8. The system according to any preceding claim, wherein the visual representation of the anatomical feature is a generated model based on medical imaging data of the anatomical feature of a patient; and/or wherein the visual representation of the anatomical feature includes virtual tissue, and the position information of the surgical device is used to apply local displacements to the virtual tissue.

9. The system according to claim 8, wherein a reaction of the virtual tissue to the applied local displacement is calculated from mechanical properties assigned to structures in the virtual tissue; preferably wherein the mechanical properties are assigned by tissue type, the tissue type including parenchyma, vasculature, bronchi, tumor, cartilage, and muscle.

10. A system for simulating thoracoscopic lung surgery, the system comprising:
a surgical device;
an imaging device configured to capture images including at least a portion of the surgical device; and
a workstation in electrical communication with at least one of the surgical device or the imaging device, the workstation including:
a display;
a processor coupled to the display; and
a memory coupled to the processor, the memory having instructions stored thereon which, when executed by the processor, cause the workstation to:
receive image data from the imaging device;
analyze the image data to determine position information of the surgical device;
generate on the display a visual representation of the surgical device relative to a visual representation of an anatomical feature based on the determined position information of the surgical device; and
simulate, on the display, an effect a manipulation of the surgical device has on the visual representation of the anatomical feature.

11. The system according to claim 10, wherein the workstation predicts the effect on the visual representation of the anatomical feature based on an analysis of the position information of the surgical device; and/or wherein the workstation predicts the effect on the visual representation of the anatomical feature based on a type of actuation of the surgical device.

12. The system according to claim 11, wherein simulating, on the display, the effect the manipulation of the surgical device has on the visual representation of the anatomical feature includes generating on the display a change in state of the visual representation of the anatomical feature.

13. The system according to any of claims 10 to 12, wherein the surgical device is a virtual representation of a surgical device.

14. A method of simulating thoracoscopic lung surgery, the method comprising:
receiving position information of a surgical device;
generating on the display a visual representation of the surgical device relative to a visual representation of an anatomical feature;
predicting an effect a manipulation of the surgical device would have on the anatomical feature; and
generating on the display a change in state of the visual representation of the anatomical feature, the change in state corresponding to the predicted effect on the anatomical feature.
